(19) Europäisches Patentamt European Patent Office Office européen des brevets

(11) **EP 4 506 118 A1**

(12) **EUROPEAN PATENT APPLICATION**
published in accordance with Art. 153(4) EPC

(43) Date of publication:
**12.02.2025 Bulletin 2025/07**

(21) Application number: **23784634.0**

(22) Date of filing: **23.03.2023**

(51) International Patent Classification (IPC):
**B25J 19/06** $^{(2006.01)}$  **A61B 34/30** $^{(2016.01)}$

(52) Cooperative Patent Classification (CPC):
**A61B 34/30; B25J 19/06**

(86) International application number:
**PCT/JP2023/011371**

(87) International publication number:
**WO 2023/195344 (12.10.2023 Gazette 2023/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.04.2022 JP 2022064718**

(71) Applicant: **Sony Group Corporation**
**Tokyo 108-0075 (JP)**

(72) Inventors:
• **SUZUKI, Hiroyuki**
**Tokyo 141-0022 (JP)**
• **TEJIMA, Asuka**
**Tokyo 141-0022 (JP)**
• **OGASAWARA, Yuki**
**Tokyo 141-0022 (JP)**

(74) Representative: **D Young & Co LLP**
**3 Noble Street**
**London EC2V 7BQ (GB)**

(54) **RETRACTION MECHANISM**

(57) A retraction mechanism according to an embodiment includes: a first member; a second member; a first structure that biases the first member toward the second member to stop the first member; a second structure that cancels the stop of the first member by the first structure according to a force applied to the first member; and a third structure that cancels the stop of the first member by the first structure according to a force applied to the second member.

FIG.1

EP 4 506 118 A1

**EP 4 506 118 A1**

## Description

Field

The present disclosure relates to a retraction mechanism.

Background

In recent years, there has been developed a technique of assigning a task requiring a large force such as transportation, a task needing fine adjustment of force such as treatment of an organ of a person, a task in a remote place as a restricted area, or the like, to an industrial robot, a medical robot, or the like, instead of a human.

Citation List

Patent Literature

Patent Literature 1: WO 2017/026167 A

Summary

Technical Problem

When a robot control system has crashed, it is important, in robot operation, to safely stop the robot and to retract its arms, surgical tools, and the like from a target. For example, at the crash of a robot tool, a remotely operated surgical robot has issues of concern such as destruction of a living tissue and a failure of the robot surgical tool. While it is necessary for a practitioner to quickly move the surgical tool away from the operative field, the conventional configuration of removing the surgical tool by an electrical command is not appropriate from the viewpoint of safety because it is implemented with intervention of robot control.
In view of this, the present disclosure proposes a retraction mechanism that enables safe retraction operation.

Solution to Problem

In order to solve the above problem, a retraction mechanism according to one embodiment of the present disclosure includes: a first member; a second member; a first structure that biases the first member toward the second member to stop the first member; a second structure that cancels the stop of the first member by the first structure according to a force applied to the first member; and a third structure that cancels the stop of the first member by the first structure according to a force applied to the second member.

Brief Description of Drawings

FIG. 1 is a schematic diagram illustrating a schematic configuration example of a retraction mechanism according to a first embodiment.
FIG. 2 is a diagram for illustrating a force applied to each portion in the configuration illustrated in FIG. 1.
FIG. 3 is a schematic view illustrating a retraction mechanism according to the first embodiment in a simplified manner.
FIG. 4 is a diagram for illustrating a motion equation of a system 1 in FIG. 3.
FIG. 5 is a diagram for illustrating a motion equation of a system 2 in FIG. 3.
FIG. 6 is a diagram for illustrating a force when active retraction and passive retraction are started in the retraction mechanism according to the first embodiment.
FIG. 7 is a diagram illustrating an example of distance dependence of an attraction force generated between magnets.
FIG. 8 is a diagram for illustrating an operation example at the time of active retraction according to the first embodiment (Part 1).
FIG. 9 is a diagram for illustrating an operation example at the time of active retraction according to the first embodiment (Part 2).
FIG. 10 is a diagram for illustrating an operation example at the time of passive retraction according to the first embodiment (Part 1).
FIG. 11 is a diagram for illustrating an operation example at the time of passive retraction according to the first

2

embodiment (Part 2).

FIG. 12 is a diagram illustrating an example of a model constructed when the retraction mechanism according to the first embodiment is analyzed by dynamics simulation.

FIG. 13 is a diagram illustrating an example of a temporal change in a tractive force of a link mechanism in the model illustrated in FIG. 12.

FIG. 14 is a diagram illustrating an example of a temporal change of a contact force between a fixed magnet and a second member when the tractive force illustrated in FIG. 13 is applied to the model illustrated in FIG. 12.

FIG. 15 is a diagram illustrating an example of a temporal change of a contact force between a biasing magnet and a second member when the tractive force illustrated in FIG. 13 is applied to the model illustrated in FIG. 12.

FIG. 16 is a diagram illustrating an example of a displacement amount of the second member when the tractive force illustrated in FIG. 13 is applied to the model illustrated in FIG. 12.

FIG. 17 is a schematic diagram illustrating a schematic configuration example of a retraction mechanism according to a second embodiment.

FIG. 18 is a schematic diagram illustrating a schematic configuration example of a retraction mechanism according to a modification of the second embodiment.

FIG. 19 is a schematic diagram illustrating a schematic configuration example of a retraction mechanism according to a third embodiment.

FIG. 20 is a schematic diagram illustrating a schematic configuration example of a retraction mechanism according to a fourth embodiment.

FIG. 21 is a diagram illustrating a specific example of a magnet according to the fourth embodiment.

FIG. 22 is a diagram illustrating another specific example of a magnet according to the fourth embodiment.

FIG. 23 is a schematic diagram illustrating a schematic configuration example of a retraction mechanism according to a fifth embodiment.

FIG. 24 is a diagram illustrating an example of a schematic configuration of an endoscopic surgery system according to the embodiment of the present disclosure.

Description of Embodiments

Embodiments of the present disclosure will be described below in detail with reference to the drawings. In each of the following embodiments, the same parts are denoted by the same reference symbols, and a repetitive description thereof will be omitted.

The present disclosure will be described in the following order.

0. Introduction
1. First embodiment
1.1 Schematic configuration example of retraction mechanism
1.2 Operating principle
1.3 Operation example at active retraction
1.4 Operation example at passive retraction
1.5 Action/effect
1.6. Operation check by dynamics simulation
2. Second embodiment
2.1 Modification
3. Third embodiment
4. Fourth embodiment
5. Fifth embodiment
6. Schematic configuration example of surgery system

0. Introduction

As described above, in the control of a machine that operates autonomously or heteronomously such as a robot or a manipulator (hereinafter, collectively referred to as a robot), it is important, when the control system has crashed, to safely stop the robot and retract a part that exerts some action on a target, such as an arm or a surgical tool (hereinafter, also referred to as an acting portion or a treatment unit) from the target. For example, in a case where a robot surgical tool has crashed in a remotely operated surgical robot, it is necessary to appropriately retract the surgical tool in a direction away from the operative field by the operation by the practitioner (also referred to as active retraction). In addition, in a case where a dangerous load is applied in a situation difficult for the practitioner to recognize, such as a case where the robot surgical tool and the biological tissue unintentionally come into contact with each other outside the field of view, it is desirable for safety to automatically retract the surgical tool (also referred to as passive retraction).

However, implementing the active retraction and the passive retraction as above by control is not appropriate from the viewpoint of safety because it is practically impossible to cope with a case where the robot has crashed in an uncontrollable state.

Therefore, in the following embodiment, a retraction mechanism capable of implementing active retraction and passive retraction without intervention of control will be described with an example.

Here, the magnitude of the force that triggers the active retraction or the passive retraction depends on the attribute of the operator and the purpose of use (also referred to as an application) of the robot. Therefore, it is difficult to achieve both types of retractions. In the aspect of an operator, while physical operating force that can be exerted by a human varies depending on sex and age, an operating force as small as 0.1 Newton (N), there is a risk of erroneous operation due to tremor. In contrast, an extremely large operating force of 10 N or more causes an ergonomic problem.

On the other hand, in the aspect of the application, the surgical tool operating force is relatively small and about 0.1 N in the application of handling fragile biological tissue such as ophthalmology or brain surgery. In contrast, a laparoscopic surgery needs a large force for an operation of lifting an organ or the like, such as a surgical tool operating force of 10 N or more.

In this manner, for the purpose of retracting the acting portion from the target, it is technically difficult to achieve both active retraction by an ergonomically appropriate operating force and passive retraction by an operating force corresponding to an external force.

Therefore, in at least some of the following embodiments, elastic bodies having mutually different characteristics are combined to make it possible to achieve both active retraction and passive retraction with different magnitudes of forces given as triggers. For example, in at least one of the following embodiments, both active retraction and passive retraction are achieved by combining nonlinear characteristics of the magnetic attraction force and linear characteristics of the spring elasticity. In addition, in at least another one of the following embodiments, an elastic body having a certain linear elastic characteristic and an elastic body having a linear elastic characteristic different from the certain elastic characteristic to achieve both active retraction and passive retraction.

Next, individual embodiments of the present disclosure will be described in detail with reference to the drawings. The following embodiment will describe an exemplary case where the retraction mechanism according to the present disclosure is provided for a surgical tool such as forceps, an electric scalpel, an injection needle, or a probe of an endoscope, which is attached to a distal end of an arm included in a medical robot such as a fundus mirror, a surgical microscope, or an endoscope system and can be remotely operated by a practitioner. However, the retraction mechanism according to the present disclosure can be provided not only for medical robots, but also for various devices that may require emergency retraction from a target, such as an industrial robot or a manipulator that performs tasks such as assembly of a product or transportation of an article in a factory or the like.

## 1. First embodiment

First, a retraction mechanism according to a first embodiment of the present disclosure will be described in detail with reference to the drawings. The present embodiment will describe an exemplary case of combining nonlinear characteristic of the magnetic attraction force and linear characteristic of the spring elasticity to achieve both active retraction and passive retraction.

### 1.1 Schematic configuration example of retraction mechanism

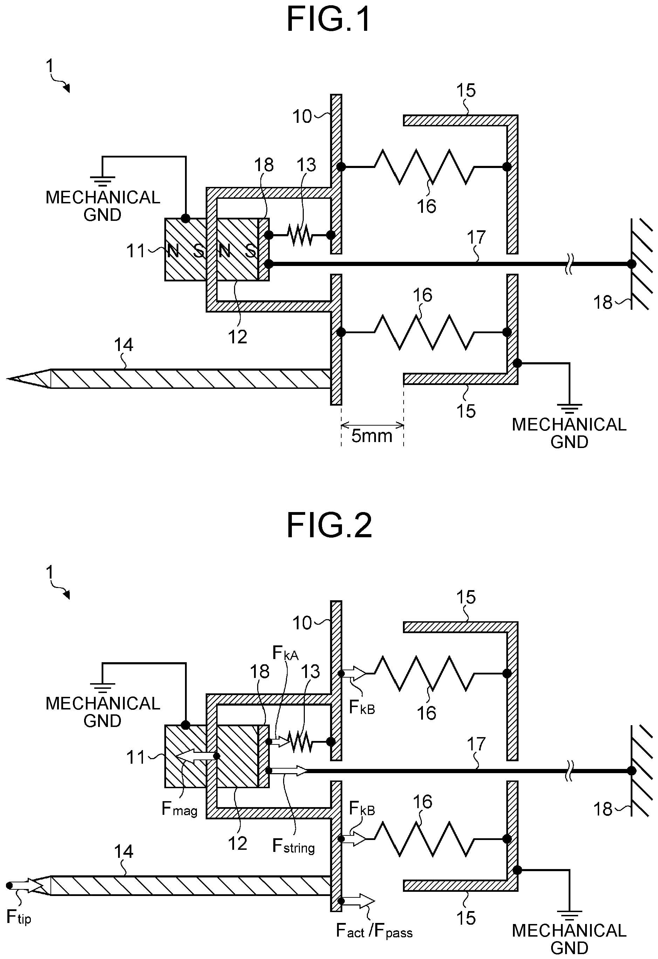

FIG. 1 is a schematic diagram illustrating a schematic configuration example of the retraction mechanism according to the present embodiment. FIG. 2 is a diagram for illustrating a force applied to each portion in the configuration illustrated in FIG. 1.

As illustrated in FIG. 1, the retraction mechanism 1 according to the present embodiment includes a first member 18, a second member 10, a third member 15, magnets 11 and 12, springs 13 and 16, a surgical tool 14, and a link mechanism 17. In the following description, in order to distinguish the magnet 11 and the magnet 12 from each other, the magnet 11 is referred to as a fixed magnet 11, and the magnet 12 is referred to as a biasing magnet 12.

In the above configuration, the fixed magnet 11 and the biasing magnet 12, constitute, for example, a first mechanism that biases the first member 18 in a first direction (direction from the biasing magnet 12 toward the fixed magnet 11). The second member 10 and the spring 13 constitute, for example, a second mechanism that biases the first member 18 in a second direction opposite to the first direction. Furthermore, the spring 16 constitutes a third mechanism that biases the first member 18 in the second direction via the second member 10 and the biasing magnet 12.

The fixed magnet 11 and the third member 15 are fixed in a system of the retraction mechanism 1 (mechanical GND). For example, the fixed magnet 11 and the third member 15 are fixed to an arm distal end of a robot on which the retraction mechanism 1 is mounted.

The second member 10 is disposed such that a position away from the third member 15 by a predetermined distance is a reference position. In other words, the second member 10 is separated from the third member 15 by a clearance distance in a state of being in contact with the fixed magnet 11.

The second member 10 is biased in a direction opposite to the fixed magnet 11 (in the right direction in the drawing) by the spring 16 having its one end fixed to the third member 15. The spring 16 may be various springs such as a coil spring and a leaf spring, for example. Furthermore, the spring 16 is not limited to a metal spring such as a coil spring or a leaf spring, and may be, for example, a rubber spring, an air spring, a liquid spring, or the like. In addition, the spring 16 may use various elastic bodies such as a diaphragm.

In addition, the surgical tool 14 for performing treatment on a patient is fixed to the second member 10. In the present example, the surgical tool 14 is a treatment unit that exerts some action on a target (patient in the present example). The direction in which the surgical tool 14 is attached to the second member 10 may be, for example, a direction (left direction in the drawing) opposite to the direction (right direction in the drawing) in which the second member 10 is biased by the spring 16. Note that, as described above, the surgical tool 14 may be a tool such as forceps, an electric scalpel, an injection needle, and a probe of an endoscope.

The biasing magnet 12 is disposed so as to face the fixed magnet 11 across the second member 10. At this time, the biasing magnet 12 is disposed such that its magnetization direction is a direction (lateral direction in the drawing) producing an attraction force with the fixed magnet 11.

The biasing magnet 12 is fixed to the first member 18. The first member 18 is biased in a direction (in a right direction in the drawing) opposite to the attracting direction between the fixed magnet 11 and the biasing magnet 12 by the spring 13 having its one end fixed to the second member 10. Similarly to the spring 16, for example, the spring 13 may use various elastic bodies such as a coil spring and a leaf spring. At this time, the elastic force of the spring 13 and the elastic force of the spring 16 may have characteristics having different gradients, or may have characteristics having a same gradient. The present embodiment has described an example in which the spring 13 and/or the spring 16 uses an elastic body having an elastic characteristic in which the magnitude of the force linearly changes according to the position of the first member 18 with respect to the fixed magnet 11. However, the configuration is not limited thereto, and it is also allowable to use an elastic body having an elastic characteristic in which the magnitude of the force nonlinearly changes.

With the above configuration, as illustrated in FIG. 2, the second member 10 receives a force (restoring force) $k_A \Delta x_A$ (force in the left direction in the drawing) to be pushed toward the fixed magnet 11 by the biasing magnet 12 and a force (restoring force) $k_B \Delta x_B$ (force in the right direction in the drawing) to be pulled toward the opposite side of the fixed magnet 11 by the spring 16. In the following description, the spring 13 is also referred to as a spring A, and the spring 16 is also referred to as a spring B. In addition, $k_A$ is a spring constant of the spring A, $k_B$ is a spring constant of the spring B, $\Delta x_A$ is a displacement amount of the spring A from its equilibrium length, and $\Delta x_B$ is a displacement amount of the spring B from its equilibrium length.

Accordingly, the force (force f11) applied to the first member 18 by the fixed magnet 11, the biasing magnet 12, and the spring A supported by the second member 10 is a total value ($f11 = f_{mag} - k_A \Delta x_A$) of an attraction force $f_{mag}$ (force in the left direction in the drawing) between the fixed magnet 11 and the biasing magnet 12 and a force $k_A \Delta x_A$ (force in the right direction in the drawing) by which the spring A pulls the first member 18.

The force (force f12) applied to the second member 10 by the fixed magnet 11, the biasing magnet 12, and the spring B supported by the third member 15 is a total value ($f12 = f_{mag} - k_B \Delta x_B$) of the attraction force $f_{mag}$ (force in the left direction in the drawing) between the fixed magnet 11 and the biasing magnet 12 and the force $k_B \Delta x_B$ (force in the right direction in the drawing) of pulling the second member 10 by the spring B.

The description returns to FIG. 1. One end of the link mechanism 17 is attached to the first member 18 that is moved by the operation of the practitioner, while the other end of the link mechanism 17 is fixed to the first member 18 to which the biasing magnet 12 is fixed. The link mechanism 17 may be formed with a thread, a wire, or a rod member, for example. However, the configuration is not limited thereto, and various modifications may be made as long as tractive force can be remotely applied to the first member 18 (more specifically, biasing magnet 12), using methods such as air pressure, hydraulic pressure, link, shape memory, soft actuator, for example.

When the tractive force $f_s$ (force in the right direction in the drawing) by the link mechanism 17 is within a range of ordinary operating force by the practitioner, in other words, when the tractive force $f_s$ by the link mechanism 17 is equal to or less than the total force f11 ($= f_{mag} - k_A \Delta x_A$) of the attraction force $f_{mag}$ between the fixed magnet 11 and the biasing magnet 12 and the restoring force $k_A \Delta x_A$ by the spring A, that is, if ($f_s \leq f_{mag} - k_A \Delta x_A$) holds, the practitioner can operate the surgical tool 14 attached to the second member 10 by operating the link mechanism 17.

1.2 Operating principle

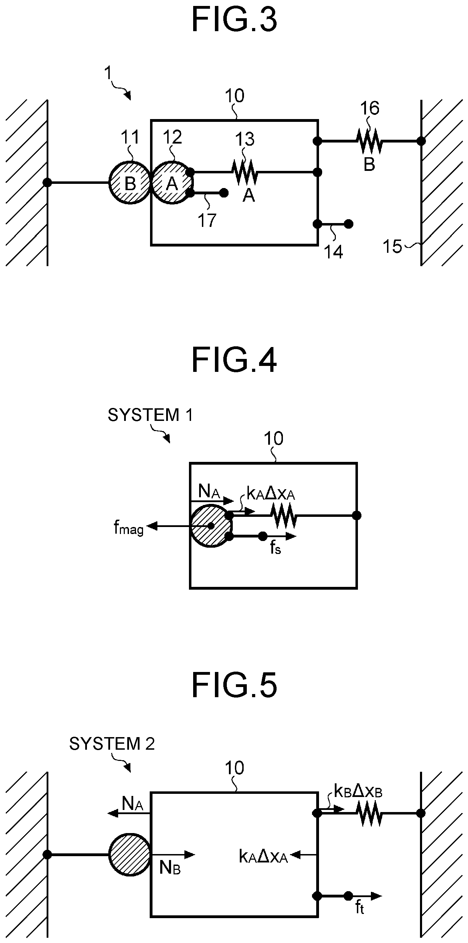

Next, an operating principle of the retraction mechanism 1 according to the present embodiment will be described. For clarity of description, the configuration of the retraction mechanism 1 according to the present embodiment is simplified as illustrated in FIG. 3.

In the simplified retraction mechanism 1 illustrated in FIG. 3, the first member 18 is omitted, and the spring 13 and the link mechanism 17 are directly attached to the biasing magnet 12. In the following description, the spring 13 is also referred to as a spring A, the spring 16 is also referred to as a spring B, the fixed magnet 11 is also referred to as a magnet B, and the biasing magnet 12 is also referred to as a magnet A.

the configuration like this includes: a system, as illustrated in FIG. 4, that starts retraction operation (active retraction operation) with the tractive force (internal force) $f_s$ of the link mechanism 17 reaching a first threshold as a trigger; and a system 2, as illustrated in FIG. 5, that starts retraction operation (passive retraction operation) with an external force $f_t$ applied to the surgical tool 14 reaching a second threshold as a trigger.

In the following description, $m_1$ may be the mass of the second component 10, $m_A$ may be the mass of the biasing magnet 12 (magnet A), $m_B$ may be the mass of the fixed magnet 11 (magnet B), $N_A$ may be the normal force that the magnet A receives from the second member 10 in a system 1 or the normal force that the second member 10 receives from the magnet B in the system 2, $N_B$ may be the normal force that the magnet A receives from the second component 10 in the system 2, $f_{mag}$ may be the magnetic force (attraction force) between the magnets A and B, $f_t$ may be the acting force (external force) at the distal end of the surgical tool 14, and $f_s$ may be the tractive force (internal force) of the link mechanism 17.

Accordingly, in the system 1 illustrated in FIG. 4, the motion equation of the magnet A (biasing magnet 12; equivalent to the first member 18) can be expressed as Formula (1) below, while in the system 2 illustrated in FIG. 5, the motion equation of the second component 10 can be expressed as Formula (2) below.

$$\begin{cases} \text{SYSTEM 1} \;:\; m_A\ddot{x}_A = f_{mag} - k_A\Delta x_A - f_s - N_A \quad (1) \\ \text{SYSTEM 2} \;:\; m_1\ddot{x}_1 = -k_B\Delta x_B - f_t - N_B + N_A + K_A\Delta x_A \quad (2) \end{cases}$$

From the above Formulas (1) and (2) as motion equations, a resting condition for both the system 1 and the system 2 to stay at rest can be obtained as the following Formulas (3) to (5).

[Mathematical Expression 2]

$$\cdot \text{ FROM RESTING CONDITION}: \ddot{x}_A = \ddot{x}_1 = 0$$

$$(1) \Leftrightarrow m_A\ddot{x}_A = f_{mag} - (k_A\Delta x_A + N_A) - f_s$$

$$(where \quad m_A\ddot{x}_A = 0)$$

$$\Leftrightarrow k_A\Delta x_A + N_A = f_{mag} - f_s \quad (3)$$

SUBSTITUTE (3) INTO (2)

$$(3) \overset{(2)}{\Leftrightarrow} m_1\ddot{x}_1 = -k_B\Delta x_B - f_t - N_B + f_{mag} - f_s$$

$$(where \quad m_1\ddot{x}_1 = 0)$$

$$\Leftrightarrow N_B = f_{mag} - k_B\Delta x_B - (f_s + f_t) \quad (4)$$

TRANSFORM (3)

$$(3) \Leftrightarrow N_A = f_{mag} - k_A\Delta x_A - f_s \quad (5)$$

On the other hand, from the above Formula (1) as a motion equation, the retraction condition for starting active retraction in the system 1 is expressed by Formula (6) below, and from the above Formula (2) as a motion equation, the retraction condition for starting passive retraction in the system 2 is expressed by Formula (7) below.

[Mathematical Expression 3]

$$\cdot \text{ FROM RETRACTION CONDITION}: N_A < 0, \quad N_B < 0$$

$$(5) \Leftrightarrow N_A = f_{mag} - k_A \Delta x_A - f_s < 0$$

$$\Leftrightarrow f_s > f_{mag} - k_A \Delta x_A \qquad (6) \quad : \text{RETRACTION CONDITION}$$
$$\text{OF SYSTEM 1}$$

$$(4) \Leftrightarrow N_B = f_{mag} - k_B \Delta x_B - (f_s + f_t)$$

$$\Leftrightarrow f_s + f_t > f_{mag} - k_B \Delta x_B \qquad (7) \quad : \text{RETRACTION CONDITION}$$
$$\text{OF SYSTEM 2}$$

$$(6) \quad : f_s > f_{mag} - k_A \Delta x_A \qquad (f_s \geq 0, \quad \Delta x_A \geq 0)$$

$$(7) \quad : f_s + f_t > f_{mag} - k_B \Delta x_B \qquad (f_t \geq 0, \quad \Delta x_B \geq 0)$$

Here, by adjusting each initial position (displacement amount from the equilibrium length at rest) of the spring A and the spring B so as to satisfy $\Delta x_A > \Delta x_B$, the force for satisfying the retraction condition of the system 1 (force being a trigger of active retraction $= f_{mag} - k_A \Delta x_a$) can be made smaller than the force for satisfying the retraction condition of the system 2 (force being a trigger of passive retraction $= f_{mag} - k_B \Delta x_B$) as in Formula (8) below.

$$f_s + f_t > f_{mag} - k_B \Delta x_B > f_s > f_{mag} - k_A \Delta x_A \qquad (8)$$

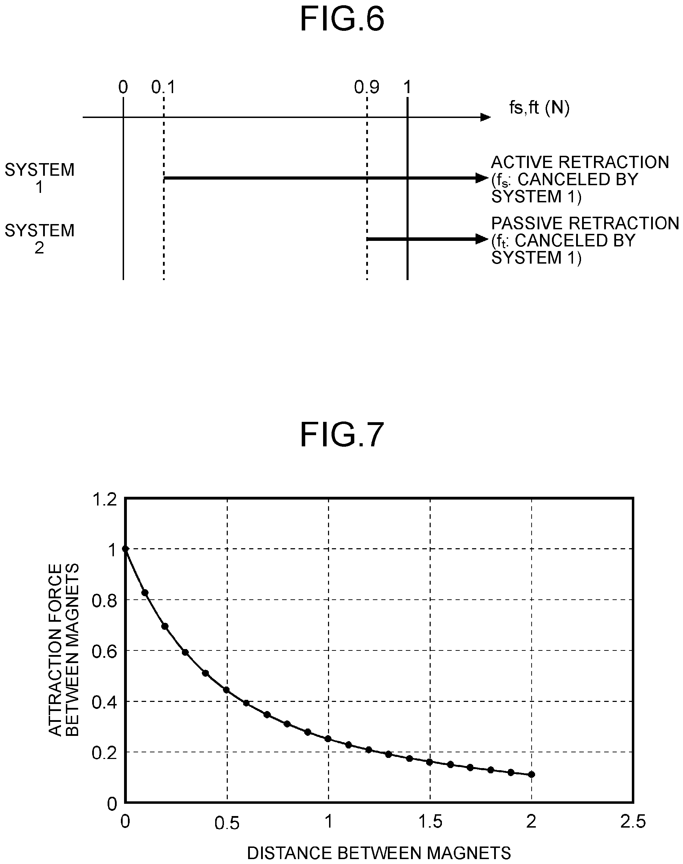

For example, in a case where the force ($= f_{mag} - k_A \Delta x_A$) as a trigger of the active retraction is designed to be 0.1 N, and the force ($= f_{mag} - k_B \Delta x_B$) as a trigger of the passive retraction is designed to be 0.9 N, as illustrated in FIG. 6, it is possible design such that the active retraction is to be executed when the tractive force $f_s$ of 0.1 N or more is applied to the link mechanism 17 and the passive retraction is to be executed when the external force $f_t$ of 0.9 N or more is applied to the surgical tool 14.

As illustrated in FIG. 7 and Formula (9) below, the attraction force $f_{mag}$ between the fixed magnet 11 and the biasing magnet 12 has a characteristic of nonlinearly changing according to the position of the biasing magnet 12 or the first member 18 with respect to the fixed magnet 11. Specifically, the attraction force $f_{mag}$ has a characteristic of decreasing exponentially (nonlinearly) in inverse proportion to the distance between the magnets. In Formula (9), K is a constant, $d_0$ is a distance between the magnets when the biasing magnet 12 comes closest to the fixed magnet 11 (corresponding to the thickness of the second member 10), and d is a distance of movement of the biasing magnet 12 in a direction away from the fixed magnet 11.

[Mathematical Expression 5]

$$\text{ATTRACTION FORCE BETWEEN MAGNETS}: f_{mag}(d) = K \frac{1}{(d_0 + d)^2} \qquad (9)$$

### 1.3 Operation example at active retraction

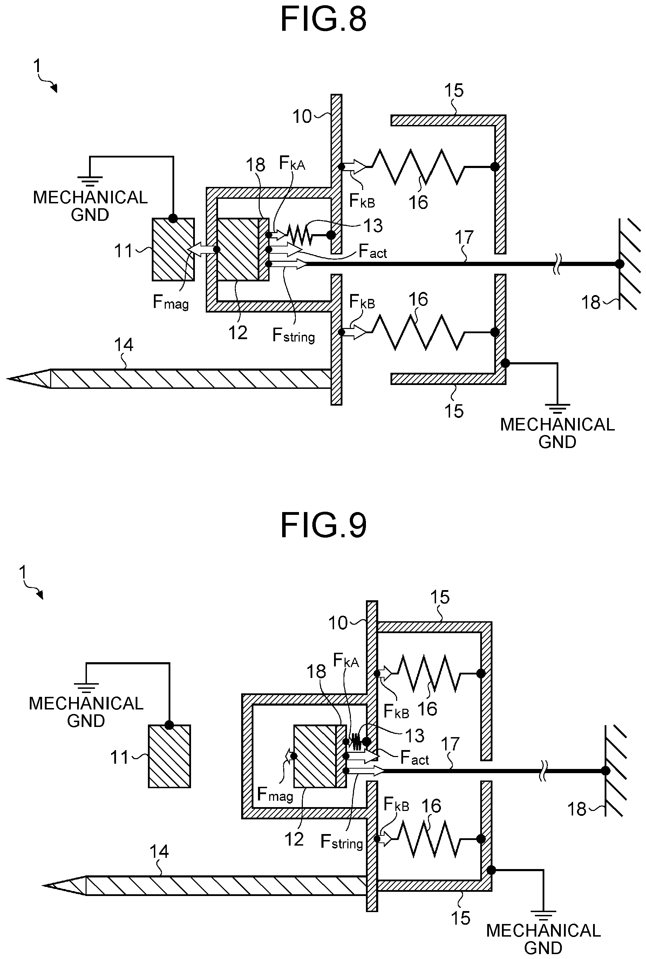

The following will describe an operation example in a case where the surgical tool 14 is automatically retracted (active retraction) in a case where an internal force (tractive force $f_s$) larger than a predetermined value is applied to the link mechanism 17. FIGS. 8 and 9 are diagrams for illustrating an operation example at the time of active retraction according to the present embodiment.

According to above Formula (6) of the retraction condition, when the tractive force $f_s$ larger than the force f11($= f_{mag} - k_A \Delta x_A$) is applied to the link mechanism 17, the first member 18 starts to move in the direction away from the fixed magnet 11. At this time, the second member 10 is pulled toward the third member 15 by the spring 16, and thus, the second member 10 moves toward the third member 15 along with the movement of the first member 18.

Subsequently, as illustrated in FIG. 9, when the biasing magnet 12 moves away from the fixed magnet 11 to a distance ($d_0 + d$) at which the attraction force $f_{mag}$ (d) between the magnets becomes smaller than the restoring force $k_A \Delta x_A$ of the spring 13, the restraint of the biasing magnet 12 by the fixed magnet 11 is released, and the first member 18 rapidly moves toward the third member 15 by the restoring force $k_A \Delta x_{BA}$ of the spring 13 and the tractive force $f_s$ of the link mechanism 17. Accordingly, the support of the second member 10 by the biasing magnet 12 is released, causing the second member 10 to rapidly move in the direction of the third member 15 by the restoring force $k_B \Delta x_B$ of the spring 16 together with the surgical tool 14 (active retraction).

Thereafter, the second member 10 moves by a clearance distance (for example, 5 mm) between the second member 10 and the third member 15, and comes into contact with the third member 15 to stop.

In this manner, in the retraction mechanism 1 according to the present embodiment, in a case where the tractive force $f_s$ larger than the predesigned force ($f11=f_{mag}-k_A\Delta x_A$) is applied to the link mechanism 17, that is, in a case where the practitioner who operates the surgical tool 14 intentionally or negligently generates the tractive force $f_s$ larger than the predesigned force ($f11=f_{mag}-k_A\Delta x_A$) on the link mechanism 17, it is possible to unlock the fixed magnet 11 and automatically retract the surgical tool 14 in a safe direction.

1.4 Operation example at passive retraction

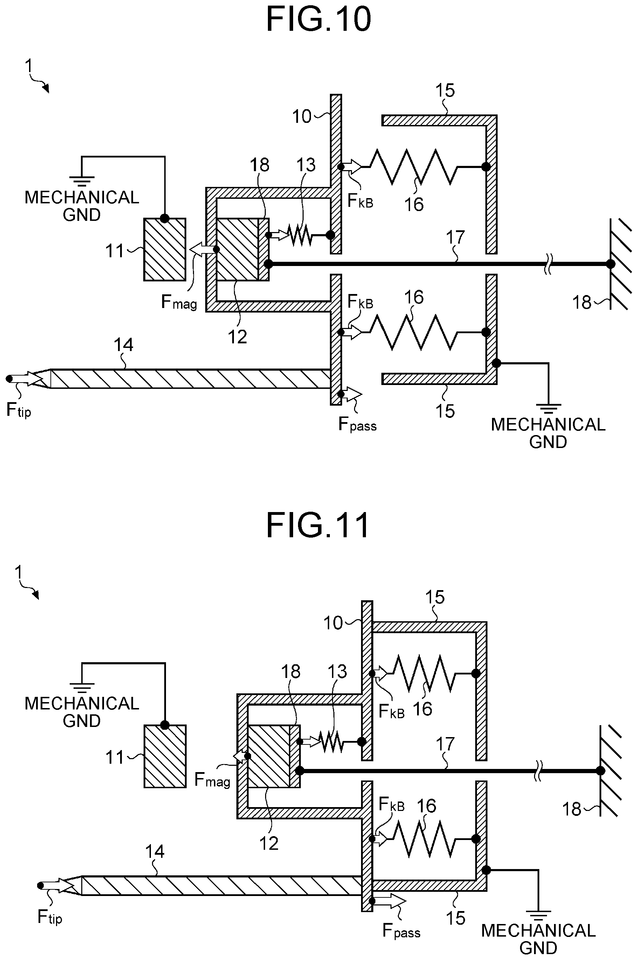

Next, the following will describe an operation example in a case where the surgical tool 14 is automatically retracted (passive retraction) in a case where the external force $f_t$ larger than a set value is applied to the surgical tool 14. FIGS. 10 and 11 are diagrams for illustrating an operation example at the time of passive retraction according to the present embodiment.

According to the above Formula (7) of the retraction condition, when a force $f_s+f_t$ larger than a force f12 ($=f_{mag}-k_B\Delta x_B$) is applied to the surgical tool 14 and the link mechanism 17, the second member 10 starts to move in the direction away from the fixed magnet 11. At this time, the biasing magnet 12 is in contact with the second member 10 by the first member 18 biased by the spring 13, and thus, the biasing magnet moves toward the third member 15 along with the movement of the second member 10.

Subsequently, as illustrated in FIG. 11, when the biasing magnet 12 moves away from the fixed magnet 11 to a distance ($d_0+d$) at which the attraction force $f_{mag}$ (d) between the magnets is smaller than the restoring force $k_B\Delta x_B$ of the spring 16, the restraint of the biasing magnet 12 by the fixed magnet 11 is released, and the second member 10 rapidly moves toward the third member 15 by the restoring force $k_B\Delta x_B$ of the spring 16 and the force $f_s+f_t$ applied to the surgical tool 14 and the link mechanism 17 (passive retraction).

Thereafter, the second member 10 moves by a clearance distance (for example, 5 mm) between the second member 10 and the third member 15, and comes into contact with the third member 15 to stop.

In this manner, in the retraction mechanism 1 according to the present embodiment, when the force $f_s+f_t$ larger than the predesigned force ($f12=f_{mag}-k_B\Delta x_B$) is applied to the surgical tool 14 and the link mechanism 17, it is possible to unlock the fixed magnet 11 to automatically retract the surgical tool 14 in a safe direction.

1.5 Action/effect

As described above, in the retraction mechanism 1 according to the present embodiment, the system 1 that implements active retraction and the system 2 that implements passive retraction are constituted as different systems. Therefore, the individual systems can be automatically executed with forces having different magnitudes as triggers. For example, it is possible to achieve both active retraction by an ergonomically appropriate operating force and passive retraction by an operating force corresponding to an external force.

In addition, since the interval between the second member 10 and the third member 15 is defined as a clearance distance, it is also possible to obtain the retraction distance of the surgical tool 14 with high reproducibility.

Furthermore, since the surgical tool 14 is operated using the link mechanism 17 in a state where the second member 10 pressed by the biasing magnet 12 is in contact with the fixed magnet 11, it is also possible to realize a backlashless mechanism.

In the above configuration, by appropriately adjusting the spring constant of the springs 13 and 16, the magnitude of the preload (length extending from the equilibrium length at the time of non-retraction), the size between the magnets 11 and 12, the magnetic force, the material, and the like of the magnets, it is possible to independently set the magnitude of the force to be a trigger of the active retraction and the passive retraction individually.

For example, in a case where the spring constant of the spring 13 is set to a large value, it is possible to configure such that passive retraction is to be executed in a case where a relatively large external force is applied to the surgical tool 14 while achieving active retraction by an ergonomically appropriate operating force. This leads to achievement of construction of a retraction mechanism suitable for a surgical robot used in operations such as laparoscopic surgery, for example. However, the property of the spring is not limited thereto, and the spring constant of the spring 13 can be set to a small value (for example, the spring constant of the spring 16 or less).

1.6. Operation check by dynamics simulation

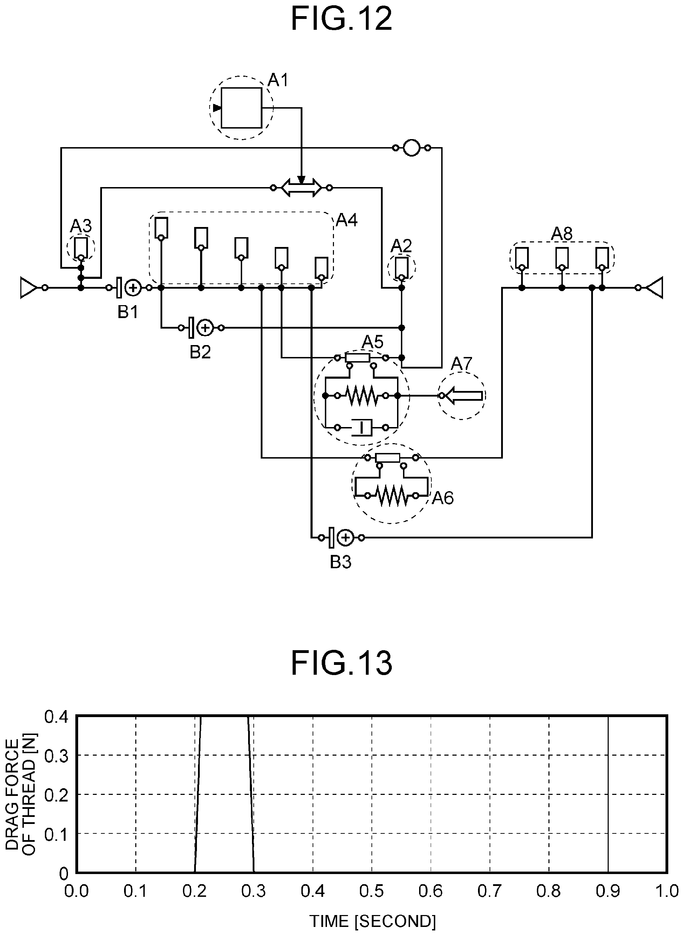

FIG. 12 is a diagram illustrating an example of a model constructed when the retraction mechanism according to the present embodiment is analyzed by dynamics simulation. In FIG. 12, an object A1 corresponds to a magnet attraction characteristic between the magnets 11 and 12, an object A2 corresponds to the fixed magnet 11, an object A3 corresponds to the biasing magnet 12, an object A4 corresponds to the second member 10, an object A5 corresponds to the spring 13, an object A6 corresponds to the spring 16, an object A7 corresponds to the tractive force exerted by the link mechanism 17, and an object A8 corresponds to the third member 15. In addition, nodes B1 to B3 each correspond to contacts between components.

In performing analysis by dynamics simulation using the model illustrated in FIG. 12, the spring constant of the spring 13 was set to 1.4 N/mm, the preload of the spring 13 was set to 0.5 mm, the spring constant of the spring 16 was set to 0.01 N/mm, the preload of the spring 16 was set to 10 mm, and the characteristics illustrated in FIG. 7 were used as the magnet attraction characteristics between the magnets 11 and 12.

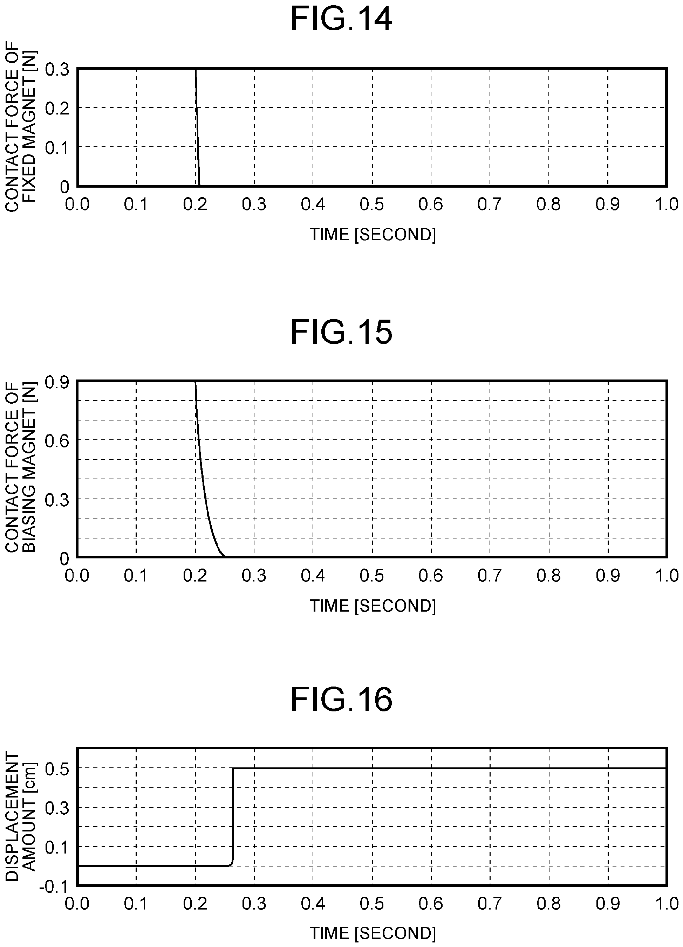

FIG. 13 is a diagram illustrating an example of a temporal change in a tractive force of a link mechanism in a model illustrated in FIG. 12. FIG. 14 is a diagram illustrating an example of a temporal change of a contact force between a fixed magnet and a second member when the tractive force illustrated in FIG. 13 is applied to the model illustrated in FIG. 12. FIG. 15 is a diagram illustrating an example of a temporal change of a contact force between a biasing magnet and a second member when the tractive force illustrated in FIG. 13 is applied to the model illustrated in FIG. 12. FIG. 16 is a diagram illustrating an example of a displacement amount of the second member when the tractive force illustrated in FIG. 13 is applied to the model illustrated in FIG. 12.

As illustrated in FIG. 13, the present simulation assumes that the tractive force $f_s$ of 0.4 N is applied with steep rising and falling during a range of 0.2 to 0.3 seconds. In this case, as illustrated in FIG. 14, the contact force between the fixed magnet 11 and the second member 10 sharply decreases with the rise of the tractive force $f_s$, while as illustrated in FIG. 15, the contact force between the fixed magnet 11 and the second member 10 gradually decreases because the second member 10 is pulled by the spring 16. As a result, as illustrated in FIG. 16, the second member 10 suddenly starts to move toward the third member 15 at a timing when the contact force between the fixed magnet 11 and the second member 10 becomes substantially zero, and after moving by a predetermined clearance distance (5 mm), the second member 10 comes into contact with the third member 15 and stops.

According to the result of dynamics simulation, it has been found that, in the retraction mechanism 1 according to the present embodiment, in a case where the tractive force $f_s$ larger than the predesigned force ($f11=f_{mag}-k_A\Delta x_A$) is applied to the link mechanism 17, that is, in a case where the practitioner who operates the surgical tool 14 intentionally or negligently generates the tractive force $f_s$ larger than the predesigned force ($f11=f_{mag}-k_A\Delta x_A$) on the link mechanism 17, it is possible to unlock the fixed magnet 11 to automatically retract the surgical tool 14 in a safe direction.

2. Second embodiment

Next, a retraction mechanism according to a second embodiment of the present disclosure will be described in detail with reference to the drawings. In the following description, the configuration, operation, and effects similar to those of the above-described embodiments will be cited, thereby omitting redundant description.

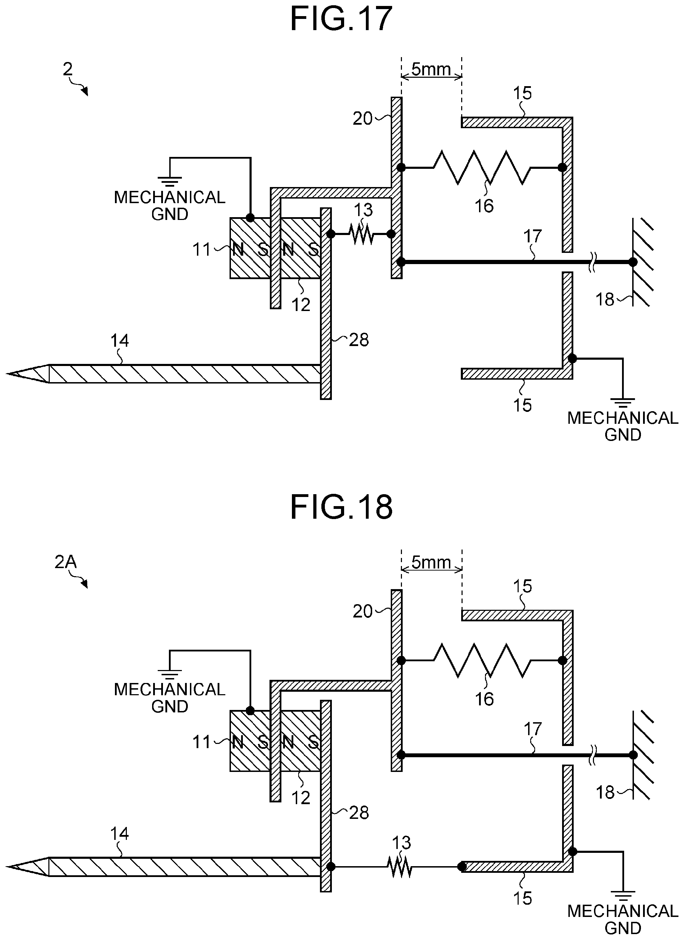

FIG. 17 is a schematic diagram illustrating a schematic configuration example of the retraction mechanism according to the present embodiment. As illustrated in FIG. 17, a retraction mechanism 2 according to the present embodiment has a configuration similar to the retraction mechanism 1 according to the first embodiment, but the first member 18 and the second member 10 are replaced with a first member 28 and a second member 20, respectively, the surgical tool 14 is fixed to the first member 28, and one end of the link mechanism 17 is attached to the second member 20. That is, in the second embodiment, the practitioner operates the surgical tool 14 by moving the second member 20 using the link mechanism 17.

In the above configuration, the fixed magnet 11 and the biasing magnet 12, for example, constitute, for example, a first mechanism that biases the first member 28 in the first direction. The second member 20 and the spring 13 constitute, for example, a second mechanism that biases the first member 28 in a second direction opposite to the first direction. Furthermore, the spring 16 constitutes a third mechanism that biases the first member 28 in the second direction via the second member 20 and the biasing magnet 12.

In this manner, in the configuration in which the surgical tool 14 is attached to the first member 28 and the link mechanism 17 is attached to the second member 20, unlike the retraction mechanism 1 according to the first embodiment, the fixed magnet 11, the biasing magnet 12, and the spring 13 constitute a mechanism for executing passive retraction in a case where an excessive load (external force $f_t$) is applied to the surgical tool 14, while the fixed magnet 11, the biasing magnet 12, and the spring 16 constitute a mechanism for executing active retraction according to the internal force (tractive force $f_s$) applied to the link mechanism 17. That is, the spring for active retraction and the spring for passive retraction are exchanged.

In the present embodiment, the motion equation of the magnet A (biasing magnet 12; equivalent to the first member 28 in the present embodiment) in the system 1 can be expressed as Formula (10) below, while the motion equation of the second member 20 in the system 2 can be expressed as Formula (11) below.

$$\begin{cases} \text{SYSTEM 1} : m_A \ddot{x}_A = -k_B \Delta x_B - f_t - N_B + N_A + K_A \Delta x_A & (10) \\ \text{SYSTEM 2} : m_1 \ddot{x}_1 = f_{mag} - k_A \Delta x_A - f_s - N_A & (11) \end{cases}$$

In this manner, in the present embodiment, the tractive force $f_s$ applied to the link mechanism 17 acts on the second component 20 in the system 2, and the external force $f_t$ applied to the surgical tool 14 acts on the magnet A (biasing magnet 12; equivalent to the first member 28) in the system 1.

Therefore, the condition when the first member 28 starts to move by the external force $f_t$ applied to the surgical tool 14 is expressed as Formula (12) below, and the condition when the second member 20 starts to move by the tractive force $f_s$ applied to the link mechanism 17 is expressed as Formula (13) below.

- **FROM RETRACTION CONDITION : $N_A < 0$, $N_B < 0$ RETRACTION CONDITION OF SYSTEM 1 :**

$$f_s + f_t > f_{mag} - k_B \Delta x_B \qquad (f_t \geq 0, \quad \Delta x_B \geq 0) \qquad (12)$$

**RETRACTION CONDITION OF SYSTEM 2**

$$f_s > f_{mag} - k_A \Delta x_A \qquad (f_s \geq 0, \quad \Delta x_A \geq 0) \qquad (13)$$

In this manner, in the retraction mechanism 2 according to the present embodiment, when the force $f_s + f_t$ larger than the force ($f21 = f_{mag} - k_B \Delta x_B$) designed in advance is applied to the surgical tool 14 and the link mechanism 17, it is possible to unlock by the fixed magnet 11 and automatically retract the surgical tool 14 in the safe direction (passive retraction).

Moreover, in the retraction mechanism 2 according to the present embodiment, when the tractive force $f_s$ larger than the predesigned force ($f22 = f_{mag} - k_A \Delta x_A$) is applied to the link mechanism 17, it is possible to unlock by the fixed magnet 11 and automatically retract the surgical tool 14 in the safe direction (active retraction).

Since other configurations, operations, and effects may be similar to those in the above-described embodiment, detailed description will be omitted here.

In the present embodiment, for example, in a case where the spring constant of the spring 13 is set to a small value, it is possible to configure such that passive retraction is to be executed in a case where a relatively small external force is applied to the surgical tool 14 while achieving active retraction by an operating force appropriate in terms of ergonomics. This configuration makes it possible to construct a retraction mechanism suitable for a surgical robot used in surgery for handling a relatively fragile living tissue such as ophthalmology or brain surgery, for example. However, the spring property is not limited thereto, and the spring constant of the spring 13 can be set to a large value (for example, the spring constant of the spring 16 or more).

2.1 Modification

The second embodiment has described an exemplary case where the spring 13 has one end attached to the first member 28 and the other end attached to the second member 20 (refer to FIG. 17). However, the configuration is not limited thereto, and for example, the other end of the spring 13 may be attached to the third member 15 as in a retraction mechanism 2A illustrated in FIG. 18. Even with this configuration, similarly to the retraction mechanism 2 illustrated in FIG. 17, the fixed magnet 11, the biasing magnet 12, and the spring 13 constitute a mechanism for executing passive retraction in a case where an excessive load (external force $f_t$) is applied to the surgical tool 14, while the fixed magnet 11, the biasing magnet 12, and the spring 16 constitute a mechanism for executing active retraction according to the internal force (tractive force $f_s$) applied to the link mechanism 17.

3. Third embodiment

Next, a retraction mechanism according to a third embodiment of the present disclosure will be described in detail with reference to the drawings. In the following description, the configuration, operation, and effects similar to those of the above-described embodiments will be cited, thereby omitting redundant description.

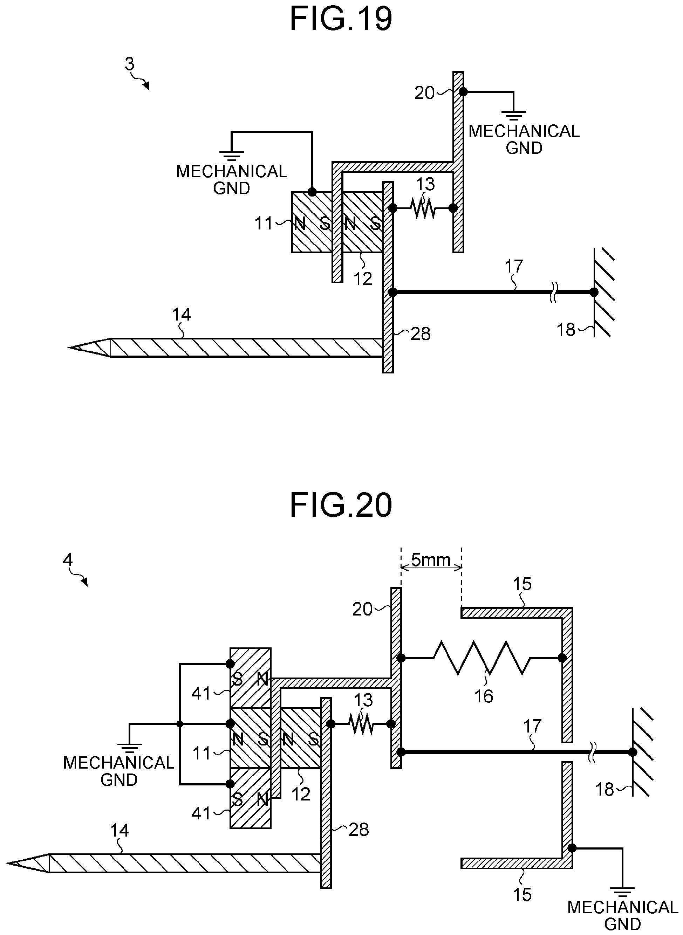

FIG. 19 is a schematic diagram illustrating a schematic configuration example of the retraction mechanism according to the present embodiment. As illustrated in FIG. 19, a retraction mechanism 3 according to the present embodiment has a configuration similar to the retraction mechanism 2 according to the second embodiment, but the third member 15 and the spring 16 are omitted, the second member 25 is fixed (mechanical GND) in the system of the retraction mechanism 3, and one end of the link mechanism 17 is attached to the first member 28.

In such a configuration, the mechanisms for executing the passive retraction and the active retraction are both constituted with the fixed magnet 11, the biasing magnet 12, and the spring 13. That is, in the present embodiment, the passive retraction and the active retraction are executed using the same mechanism. Therefore, in the present embodiment, the condition when the first member 28 starts to move by the external force $f_t$ applied to the surgical tool 14 and the condition when the first member 28 starts to move by the tractive force $f_s$ applied to the link mechanism 17 are both the same condition.

In the present embodiment, execution of the passive retraction and the active retraction uses the common mechanism, the magnitude of the force being a trigger at the execution of the passive retraction and the active retraction is the same.

Since other configurations, operations, and effects may be similar to those in the above-described embodiment, detailed description will be omitted here.

## 4. Fourth embodiment

Next, a retraction mechanism according to a fourth embodiment of the present disclosure will be described in detail with reference to the drawings. In the following description, the configuration, operation, and effects similar to those of the above-described embodiments will be cited, thereby omitting redundant description. The following will describe an exemplary case where the retraction mechanism 2 according to the second embodiment is used as a base. However, the configuration is not limited thereto, and a retraction mechanism according to another embodiment or a modification thereof may be used as the base.

FIG. 20 is a schematic diagram illustrating a schematic configuration example of the retraction mechanism according to the present embodiment. As illustrated in FIG. 20, the retraction mechanism 4 according to the present embodiment has a configuration similar to the retraction mechanism 2 according to the second embodiment, but a magnet 41 for suppressing lateral displacement of the biasing magnet 12 is fixed (as mechanical GND) around the fixed magnet 11 facing the biasing magnet 12.

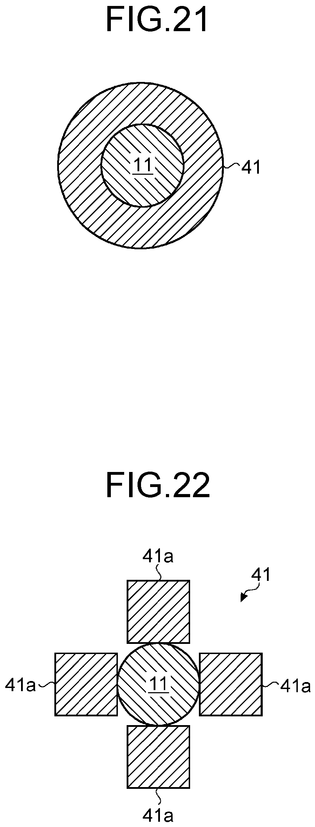

FIGS. 21 and 22 are diagrams for illustrating a specific example of the magnet according to the present embodiment. As illustrated in FIG. 21, the magnet 41 may have, for example, a ring shape, and the fixed magnet 11 may be accommodated in a center hole thereof. As illustrated in FIG. 22, the magnet 41 may include a plurality of magnets 41a disposed so as to surround the fixed magnet 11.

The magnet 41 is disposed to set its magnetization direction to be opposite to the magnetization direction of the fixed magnet 11. With this arrangement, even when the biasing magnet 12 is displaced from a region facing the fixed magnet 11 to the upper, lower, left, or right direction, the magnet 41 and the biasing magnet 12 repel each other, making it possible to suppress displacement of the biasing magnet 12 from the region facing the fixed magnet 11.

Since other configurations, operations, and effects may be similar to those in the above-described embodiment, detailed description will be omitted here.

## 5. Fifth embodiment

Next, a retraction mechanism according to a fifth embodiment of the present disclosure will be described in detail with reference to the drawings. In the following description, the configuration, operation, and effects similar to those of the above-described embodiments will be cited, thereby omitting redundant description. The following will describe an exemplary case where the retraction mechanism 2 according to the second embodiment is used as a base. However, the configuration is not limited thereto, and a retraction mechanism according to another embodiment or a modification thereof may be used as the base.

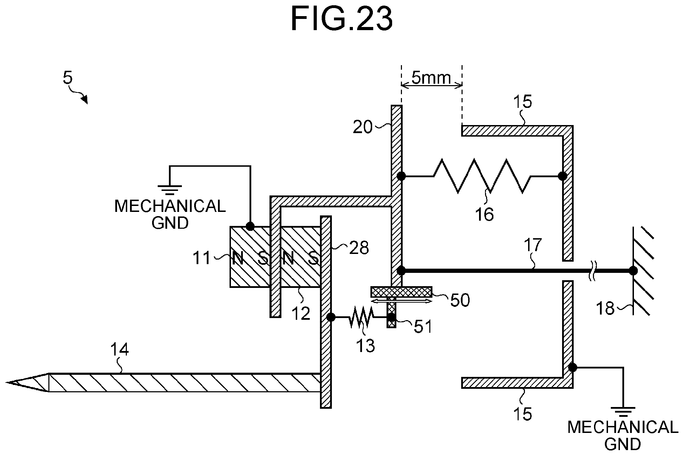

FIG. 23 is a schematic diagram illustrating a schematic configuration example of the retraction mechanism according to the present embodiment. As illustrated in FIG. 23, the retraction mechanism 5 according to the present embodiment has a configuration similar to the retraction mechanism 2 according to the second embodiment, but an adjustment mechanism 50 is provided in the second member 20 and one end of the spring 13 is attached to a slider 51 of the adjustment mechanism 50.

The adjustment mechanism 50 includes a rail extending in an arrangement direction of the fixed magnet 11 and the biasing magnet 12. The slider 51 is slidably attached to the rail. Accordingly, by moving the slider 51 along the rail, it is possible to adjust the preload of the spring 13, that is, the restoring force $k_A \Delta x_A$ of the spring 13 in the non-retracted state. As a result, it is possible to adjust the magnitude of the force (tractive force $f_s$) being a trigger of the system constituted with the fixed magnet 11, the biasing magnet 12, and the spring 13, that is, the mechanism for active retraction.

Since other configurations, operations, and effects may be similar to those in the above-described embodiment, detailed description will be omitted here.

6. Schematic configuration example of surgery system

Next, an example of a surgery system to which the retraction mechanism according to the embodiment above can be applied will be described in detail with reference to the drawings. The following description will describe, as an example, an endoscopic surgery system as a system to which the retraction mechanism can be applied. However, the application target is not limited thereto, and the system can be various systems that may require retraction of the acting portion from the target.

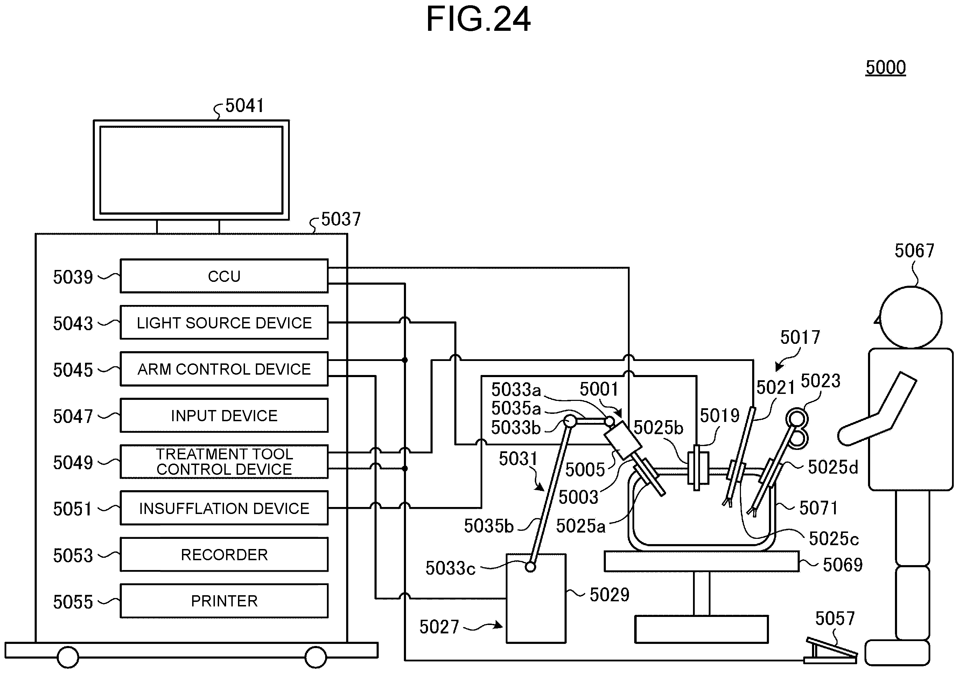

FIG. 24 is a diagram illustrating an example of a schematic configuration of an endoscopic surgery system according to the present disclosure. FIG. 24 illustrates a scene in which a surgeon 5067 is performing surgery on a patient 5071 on a patient bed 5069 using an endoscopic surgery system 5000. As illustrated in FIG. 24, the endoscopic surgery system 5000 includes: an endoscope 5001, other surgical tools 5017; a support arm unit 5027 that supports the endoscope 5001; and a cart 5037 equipped with various devices for endoscopic surgery. Hereinafter, details of the endoscopic surgery system 5000 will be sequentially described.

(Surgical tool 5017)

In endoscopic surgery, an abdominal wall is punctured with a plurality of tubular laparotomy instruments referred to as trocars 5025a to 5025d, for example, instead of a method of cutting the abdominal wall for open surgery. Through the trocars 5025a to 5025d, a lens barrel 5003 of the endoscope 5001 and other surgical tools 5017 are inserted into the body cavity of the patient 5071. In the example of FIG. 24, as other surgical tools 5017, an insufflation tube 5019, an energy treatment tool 5021 and forceps 5023 are being inserted into the body cavity of the patient 5071. Furthermore, the energy treatment tool 5021 is a treatment tool used for incision and detachment of tissues, blood vessel sealing, or the like, by using highfrequency current or ultrasonic vibration. The surgical tool 5017 illustrated in FIG. 24 is just an example, and other applicable examples of the surgical tool 5017 include various surgical tools generally used in endoscopic surgery, such as tweezers and a retractor.

(Support arm unit 5027)

The support arm unit 5027 includes an arm unit 5031 extending from a base unit 5029. In the example illustrated in FIG. 24, the arm unit 5031 includes joints 5033a, 5033b, and 5033c and links 5035a and 5035b, and is driven under the control of an arm control device 5045. The arm unit 5031 supports the endoscope 5001 and controls the position and posture of the endoscope 5001. This makes it possible to stabilize the position of the endoscope 5001.

(Endoscope 5001)

The endoscope 5001 includes a lens barrel 5003 having a region of a predetermined length from a distal end thereof to be inserted into a body cavity of the patient 5071, and a camera head 5005 connected to a proximal end of the lens barrel 5003. The example illustrated in FIG. 24 illustrates the endoscope 5001 as a rigid endoscope having the lens barrel 5003 of a rigid type. However, the endoscope 5001 is not particularly limited in the embodiment of the present disclosure and can be a flexible endoscope having the lens barrel 5003 of a flexible material.

The distal end of the lens barrel 5003 has an aperture to which an objective lens is fitted. The endoscope 5001 is connected to a light source device 5043, such that light generated by the light source device 5043 is introduced to a distal end of the lens barrel 5003 by a light guide extending in the inside of the lens barrel 5003 and is emitted toward an observation target in a body cavity (abdominal cavity) of the patient 5071 through the objective lens. In the embodiment of the present disclosure, the endoscope 5001 may be a front direct view mirror or an oblique view mirror, and is not particularly limited.

An optical system and an imaging element are provided inside the camera head 5005. Reflected light (observation light) from the observation target is focused on the imaging element by the optical system. The observation light is photo-electrically converted by the imaging element to generate an electric signal corresponding to the observation light, namely, a pixel signal corresponding to an observation image. The pixel signal is transmitted as RAW data to a Camera Control Unit (CCU) 5039. The camera head 5005 has a function of adjusting a magnification and a focal length by appropriately driving the optical system.

Incidentally, the camera head 5005 may include a plurality of imaging elements in order to support 3D viewing (stereoscopic viewing) or the like. In this case, a plurality of relay optical systems is to be provided inside the lens barrel 5003 in order to guide the observation light to each of the plurality of imaging elements. Furthermore, in the embodiment of the present disclosure, different types of imaging elements can be provided, which will be described below. Furthermore, details of the camera head 5005 and the lens barrel 5003 according to the embodiment of the present disclosure will also be described below.

(Various devices mounted on cart)

First, under the control of the CCU 5039, a display device 5041 displays an image based on the pixel signal that has undergone the image processing performed by the CCU 5039. When the endoscope 5001 is a device compatible with high-resolution imaging such as 4K (the number of horizontal pixels 3840 $\times$ the number of vertical pixels 2160) or 8K (the number of horizontal pixels 7680 $\times$ the number of vertical pixels 4320), and/or when the endoscope 5001 is a device compatible with 3D display, for example, the display device 5041 is to be a display device capable of high-resolution display and/or capable of 3D display, corresponding to individual specs. Furthermore, the display device 5041 may be provided in plurality, each having different resolutions and sizes for different applications.

An image of the surgical site in the body cavity of the patient 5071 captured by the endoscope 5001 is displayed on the display device 5041. While viewing the surgical site image displayed on the display device 5041 in real time, the surgeon 5067 can perform procedures such as resecting the affected part by using the energy treatment tool 5021 and the forceps 5023. Although not illustrated, the insufflation tube 5019, the energy treatment tool 5021, and the forceps 5023 are supported by a person such as the surgeon 5067 and assistants, for example, during the surgery.

The CCU 5039 includes a central processing unit (CPU), a graphics processing unit (GPU) or the like and cab comprehensively control operation of the endoscope 5001 and the display device 5041. Specifically, the CCU 5039 applies, on the pixel signal received from the camera head 5005, various types of image processing for displaying an image based on the pixel signal, such as developing processing (demosaicing). The CCU 5039 provides the pixel signal that has undergone the image processing to the display device 5041. Furthermore, the CCU 5039 transmits a control signal to the camera head 5005 and controls driving thereof. The control signal can include information regarding imaging conditions such as magnification and focal length. Note that details of the CCU5039 according to the embodiment of the present disclosure will be described below.

The light source device 5043 includes a light source such as, for example, a Light Emitting Diode (LED) and supplies irradiation light upon imaging of a surgical site to the endoscope 5001. Details of the light source device 5043 according to the embodiment of the present disclosure will be described below.

The arm control device 5045 includes, for example, a processor such as a CPU, and operates according to a predetermined program to control drive of the arm unit 5031 of the support arm unit 5027 according to a predetermined control method. Note that details of the arm control device 5045 according to the embodiment of the present disclosure will be described below.

An input device 5047 is an input interface for the endoscopic surgery system 5000. The surgeon 5067 can input various types of information and input instructions to the endoscopic surgery system 5000 via the input device 5047. For example, the surgeon 5067 inputs various types of information related to the surgery, such as physical information regarding the patient and information regarding the surgical procedure, via the input device 5047. Furthermore, the surgeon 5067 can input, through the input device 5047, an instruction to drive the arm unit 5031, an instruction to change imaging conditions (type of irradiation light, magnification, focal length, or the like) of the endoscope 5001, and an instruction to drive the energy treatment tool 5021, for example. The type of the input device 5047 is not limited, and the input device 5047 may be various known input devices. Examples of applicable input devices 5047 include a mouse, a keyboard, a touch panel, a switch, a foot switch 5057, and/or a lever. When a touch panel is used as the input device 5047, for example, the touch panel may be provided on a display surface of the display device 5041.

Alternatively, the input device 5047 may be a device worn on a part of the body of the surgeon 5067, such as an eyeglass-type wearable device or a head mounted display (HMD). In this case, various inputs are performed according to the gesture or the line of sight of the surgeon 5067 detected by these devices. Furthermore, the input device 5047 can include a camera capable of detecting movement of the surgeon 5067, and various inputs may be performed according to a gesture or a line of sight of the surgeon 5067 detected from an image captured by the camera. Furthermore, the input device 5047 includes a microphone capable of capturing voice of the surgeon 5067, and various inputs are performed by voice through the microphone. In this manner, with a configuration of the input device 5047 capable of inputting various types of information in a noncontact manner, it is possible for the user (for example, the surgeon 5067) located in a clean area to perform noncontact operation of a device located in an unclean area. In addition, since the surgeon 5067 can operate the device without releasing a hand from one's surgical tool, leading to enhancement of convenience for the surgeon 5067.

A treatment tool control device 5049 controls the drive of the energy treatment tool 5021 for ablation or dissection of tissue, sealing of blood vessels, and the like. An insufflation device 5051 feeds gas into a body cavity of the patient 5071 through the insufflation tube 5019 to inflate the body cavity in order to secure the visual field of the endoscope 5001 and secure the working space for the surgeon 5067. A recorder 5053 is a device capable of recording various types of information associated with surgery. A printer 5055 is a device capable of printing various types of information associated with surgery in various forms such as text, image, graph, or the like.

Furthermore, an example of a detailed configuration of the support arm unit 5027 will be described. The support arm unit 5027 includes the base unit 5029 which is a pedestal, and the arm unit 5031 extending from the base unit 5029. In the example illustrated in FIG. 24, the arm unit 5031 is formed with the plurality of joints 5033a, 5033b, and 5033c and the plurality of links 5035a and 5035b coupled via the joints 5033b. However, for the sake of simplicity, FIG. 24 illustrates the configuration of the arm unit 5031 in a simplified manner. Specifically, the shapes, the number and the arrangement of the joints 5033a to 5033c and the links 5035a and 5035b, the directions of the rotation axes of the joints 5033a to 5033c, or the like, can be appropriately set so that the arm unit 5031 has a desired degree of freedom. For example, the arm unit 5031 can be suitably configured to have six degrees of freedom, or more. With this configuration, the endoscope 5001 can be freely moved within the movable range of the arm unit 5031, making it possible to insert the lens barrel 5003 of the endoscope 5001 into the body cavity of the patient 5071 from a desired direction.

Each of the joints 5033a to 5033c is equipped with an actuator. Each of the joints 5033a to 5033c is rotatable about a predetermined rotation axis by the drive of the actuator. The drive of the actuator is controlled by the arm control device 5045, thereby controlling the rotation angle of each of the joints 5033a to 5033c and controlling the drive of the arm unit 5031. This control can achieve the control of the position and posture of the endoscope 5001. At this time, the arm control device 5045 can control the drive of the arm unit 5031 by various known control methods such as force control or position control.

For example, the surgeon 5067 may appropriately perform an operation input via the input device 5047 (including the foot switch 5057) so as to appropriately control the drive of the arm unit 5031 by the arm control device 5045 in accordance with the operation input, leading to the control of the position and posture of the endoscope 5001. Incidentally, the arm unit 5031 may be operated by a method referred to as a master-slave method. In this case, the arm unit 5031 (slave) can be remotely operated by the surgeon 5067 via the input device 5047 (master console) installed at a place away from the operating room or in the operating room.

Here, the endoscope 5001 is typically supported by a doctor as an endoscopist in endoscopic surgery. In contrast, in the embodiment of the present disclosure, the use of the support arm unit 5027 makes it possible to reliably secure the position of the endoscope 5001 without manual work, leading to stable acquisition of an image of the surgical site and smooth execution of surgery.

Note that the arm control device 5045 does not necessarily have to be provided in the cart 5037. Furthermore, the arm control device 5045 does not necessarily have to be one device. For example, the arm control device 5045 may be provided in each of the joints 5033a to 5033c of the arm unit 5031 of the support arm unit 5027, and the plurality of arm control devices 5045 may cooperate with each other to achieve the drive control of the arm unit 5031.

Next, an example of a detailed configuration of the light source device 5043 will be described. The light source device 5043 supplies the endoscope 5001 with irradiation light for imaging the surgical site. The light source device 5043 is formed with, for example, an LED, a laser light source, or a white light source constituted by a combination of these. At this time, in a case where the white light source is constituted with the combination of RGB laser light sources, it is possible to control the output intensity and the output timing of individual colors (individual wavelengths) with high accuracy. Accordingly, it is possible to perform white balance adjustment of the captured image on the light source device 5043. Furthermore, in this case, by emitting the laser light from each of the RGB laser light sources to an observation target on the time-division basis and by controlling the drive of the imaging element of the camera head 5005 in synchronization with the light emission timing, it is also possible to capture the image corresponding to each of RGB colors on the time-division basis. According to this method, a color image can be obtained even if color filters are not provided for the imaging element.

Furthermore, the drive of the light source device 5043 may be controlled so as to change the intensity of the output light at predetermined time intervals. With the control of the drive of the imaging element of the camera head 5005 in synchronization with the timing of the change of the intensity of the light so as to obtain images on the time-division basis and combine the images, it is possible to generate an image with high dynamic range without a state such as blackout shadows or blown out highlights (overexposure).

Furthermore, the light source device 5043 may be configured to be able to supply light in a predetermined wavelength band corresponding to special light observation. The special light observation is used to perform narrowband light observation (narrow band imaging). The narrowband light observation uses the wavelength dependency of the light absorption in the body tissue and emits light in a narrower band compared with the irradiation light (that is, white light) at ordinary observation, thereby imaging a predetermined tissue such as a blood vessel of the mucosal surface layer with high contrast. Alternatively, the special light observation may include fluorescent observation for obtaining an image from fluorescent light generated by emission of excitation light. Fluorescence observation can be performed to observe fluorescence emitted from a body tissue to which excitation light is applied (autofluorescence observation), and can be performed with local administration of reagent such as indocyanine green (ICG) to the body tissue, and together with this, excitation light corresponding to the fluorescence wavelength of the reagent is emitted to the body tissue to obtain a fluorescent image, or the like. The light source device 5043 can be configured to be able to supply narrow band light and/or excitation light corresponding to such special light observation.

The retraction mechanism according to the above-described embodiment may be applied to the lens barrel 5003 of the endoscope 5001, the insufflation tube 5019, the energy treatment tool 5021, the forceps 5023, and the like in the endoscopic surgery system 5000 illustrated in FIG. 24.

The embodiments of the present disclosure have been described above. However, the technical scope of the present disclosure is not limited to the above-described embodiments, and various modifications can be made without departing from the scope of the present disclosure. Moreover, it is allowable to combine the components across different embodiments and modifications as appropriate.

The effects described in individual embodiments of the present specification are merely examples, and thus, there may be other effects, not limited to the exemplified effects.

Note that the present technique can also have the following configurations.

(1) A retraction mechanism comprising:

a first member;
a second member;
a first structure that biases the first member toward the second member to stop the first member;
a second structure that cancels the stop of the first member by the first structure according to a force applied to the first member; and
a third structure that cancels the stop of the first member by the first structure according to a force applied to the second member.

(2) The retraction mechanism according to (1),

wherein the first structure biases the first member in a first direction toward the second member,
the second structure biases the first member in a second direction opposite to the first direction, and
the third structure biases the first member in the second direction.

(3) The retraction mechanism according to (2),
wherein a first characteristic in which the first structure generates a first force that biases the first member in the first direction according to a position of the first member is different from a second characteristic in which the second structure generates a second force that biases the first member in the second direction according to the position of the first member and from a third characteristic in which the third structure generates a third force that biases the first member in the second direction according to the position of the first member.

(4) The retraction mechanism according to (3),

wherein the first characteristic is a characteristic in which a magnitude of the first force nonlinearly changes according to the position of the first member, and
at least one of the second characteristic and the third characteristic is a characteristic in which a magnitude of the second force or the third force linearly changes according to the position of the first member.

(5) The retraction mechanism according to any one of (2) to (4),
wherein the first structure includes:

a first magnet being a fixed magnet; and
a second magnet movable in the first direction with respect to the first magnet, and
the second magnet is fixed to the first member.

(6) The retraction mechanism according to any one of (2) to (5),

wherein the second structure includes
a first elastic body that biases the first member in the second direction, and
the third structure includes a second elastic body that biases the second member in the second direction.

(7) The retraction mechanism according to (6),
wherein at least one of the first elastic body and the second elastic body is a spring or a diaphragm.
(8) The retraction mechanism according to any one of (2) to (7),
wherein the first structure includes:

a first magnet being a fixed magnet; and
a second magnet movable in the first direction with respect to the first magnet,
the first member holds the second magnet,

the second structure includes:

a second member partially sandwiched between the first magnet and the second magnet; and
a first elastic body having one end attached to the first member and the other end attached to the second member so as to bias the first member in the second direction, and
the third structure includes a second elastic body having one end attached to the second member and the other end fixed so as to bias the second member in the second direction.

(9) The retraction mechanism according to any one of (2) to (8),
wherein the second structure includes a structure of exerting traction on the first member in the second direction according to an operation by a user.
(10) The retraction mechanism according to (6) or (7), further comprising

a treatment unit attached to the second member,
wherein the second structure includes a structure of exerting traction on the first member in the second direction according to an operation by a user.

(11) The retraction mechanism according to (6) or (7),
wherein the third structure includes a structure of exerting traction on the second member in the second direction according to an operation by a user.
(12) The retraction mechanism according to (11), further comprising
a treatment unit attached to the first member.
(13) The retraction mechanism according to (5), further comprising
a third magnet arranged to repel the second magnet in a third direction perpendicular to the first direction with respect to the first magnet.
(14) The retraction mechanism according to any one of (2) to (13), further comprising
an adjustment mechanism that adjusts the force with which the second structure biases the first member in the second direction.
(15) The retraction mechanism according to (8), further comprising
an adjustment mechanism that adjusts an attachment position of the other end of the first elastic body to the second member, the adjustment performed in the second direction.
(16) The retraction mechanism according to (1) or (2),
wherein the second structure and the third structure are identical.

Reference Signs List

1, 2, 2A, 3, 4, 5 RETRACTION MECHANISM
10, 20 SECOND MEMBER
11 FIXED MAGNET
12 BIASING MAGNET
13, 16 SPRING
14 SURGICAL TOOL
15 THIRD MEMBER
17 LINK MECHANISM
18, 28 FIRST MEMBER
41, 41a MAGNET
50 ADJUSTMENT MECHANISM
51 SLIDER

**Claims**

1. A retraction mechanism comprising:

a first member;
a second member;

a first structure that biases the first member toward the second member to stop the first member;
a second structure that cancels the stop of the first member by the first structure according to a force applied to the first member; and
a third structure that cancels the stop of the first member by the first structure according to a force applied to the second member.

2. The retraction mechanism according to claim 1,

wherein the first structure biases the first member in a first direction toward the second member,
the second structure biases the first member in a second direction opposite to the first direction, and
the third structure biases the first member in the second direction.

3. The retraction mechanism according to claim 2,
wherein a first characteristic in which the first structure generates a first force that biases the first member in the first direction according to a position of the first member is different from a second characteristic in which the second structure generates a second force that biases the first member in the second direction according to the position of the first member and from a third characteristic in which the third structure generates a third force that biases the first member in the second direction according to the position of the first member.

4. The retraction mechanism according to claim 3,

wherein the first characteristic is a characteristic in which a magnitude of the first force nonlinearly changes according to the position of the first member, and
at least one of the second characteristic and the third characteristic is a characteristic in which a magnitude of the second force or the third force linearly changes according to the position of the first member.

5. The retraction mechanism according to claim 2,
wherein the first structure includes:

a first magnet being a fixed magnet; and
a second magnet movable in the first direction with respect to the first magnet, and
the second magnet is fixed to the first member.

6. The retraction mechanism according to claim 2,

wherein the second structure includes
a first elastic body that biases the first member in the second direction, and
the third structure includes a second elastic body that biases the second member in the second direction.

7. The retraction mechanism according to claim 6,
wherein at least one of the first elastic body and the second elastic body is a spring or a diaphragm.

8. The retraction mechanism according to claim 2,
wherein the first structure includes:

a first magnet being a fixed magnet; and
a second magnet movable in the first direction with respect to the first magnet,
the first member holds the second magnet,
the second structure includes:

a second member partially sandwiched between the first magnet and the second magnet; and
a first elastic body having one end attached to the first member and the other end attached to the second member so as to bias the first member in the second direction, and
the third structure includes a second elastic body having one end attached to the second member and the other end fixed so as to bias the second member in the second direction.

9. The retraction mechanism according to claim 2,
wherein the second structure includes a structure of exerting traction on the first member in the second direction according to an operation by a user.

10. The retraction mechanism according to claim 6, further comprising

a treatment unit attached to the second member,
wherein the second structure includes a structure of exerting traction on the first member in the second direction according to an operation by a user.

11. The retraction mechanism according to claim 6,
wherein the third structure includes a structure of exerting traction on the second member in the second direction according to an operation by a user.

12. The retraction mechanism according to claim 11, further comprising
a treatment unit attached to the first member.

13. The retraction mechanism according to claim 5, further comprising
a third magnet arranged to repel the second magnet in a third direction perpendicular to the first direction with respect to the first magnet.

14. The retraction mechanism according to claim 2, further comprising
an adjustment mechanism that adjusts the force with which the second structure biases the first member in the second direction.

15. The retraction mechanism according to claim 8, further comprising
an adjustment mechanism that adjusts an attachment position of the other end of the first elastic body to the second member, the adjustment performed in the second direction.

16. The retraction mechanism according to claim 1,
wherein the second structure and the third structure are identical.

# FIG.1

# FIG.2

# FIG.3

# FIG.4

SYSTEM 1

# FIG.5

SYSTEM 2

# FIG.6

# FIG.7

DISTANCE BETWEEN MAGNETS

# FIG.8

# FIG.9

# FIG.10

# FIG.11

# FIG.12

# FIG.13

## FIG.14

## FIG.15

## FIG.16

# FIG.17

# FIG.18

# FIG.19

3

11 N S N S
12
13
20
MECHANICAL GND
MECHANICAL GND
14
17
28
18

# FIG.20

4

5mm

20
15
16
41 S N
11 N S N S
13
17
41 S N
12
MECHANICAL GND
18
14
28
15
MECHANICAL GND

# FIG.21

# FIG.22

# FIG.23

# FIG.24

## INTERNATIONAL SEARCH REPORT

| International application No. |
|---|
| **PCT/JP2023/011371** |

**A. CLASSIFICATION OF SUBJECT MATTER**

***B25J 19/06***(2006.01)i; ***A61B 34/30***(2016.01)i
FI: A61B34/30; B25J19/06

According to International Patent Classification (IPC) or to both national classification and IPC

**B. FIELDS SEARCHED**

Minimum documentation searched (classification system followed by classification symbols)

B25J19/06; A61B34/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2003-93403 A (OLYMPUS OPTICAL CO., LTD.) 02 April 2003 (2003-04-02) paragraphs [0056]-[0066], fig. 14, 15 | 1, 16 |
| A | | 2-15 |
| X | CD-ROM of the specification and drawings annexed to the request of Japanese Utility Model Application No. 44340/1987 (Laid-open No. 154187/1988) (HOYA CORP.) 11 October 1988 (1988-10-11), page 5, line 3 to page 7, line 15, fig. 1, 2 | 1-2, 16 |
| A | | 3-15 |
| X | Microfilm of the specification and drawings annexed to the request of Japanese Utility Model Application No. 26694/1985 (Laid-open No. 142953/1986) (KOBE STEEL, LTD.) 03 September 1986 (1986-09-03), page 3, line 13, page 6, line 2, fig. 1 | 1-2, 16 |
| A | | 3-15 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 May 2023** | **16 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

| INTERNATIONAL SEARCH REPORT | International application No. |
|---|---|
| Information on patent family members | PCT/JP2023/011371 |

| Patent document cited in search report | | | Publication date (day/month/year) | Patent family member(s) | | | Publication date (day/month/year) |
|---|---|---|---|---|---|---|---|
| JP | 2003-93403 | A | 02 April 2003 | JP | 7-194610 | A | |
| | | | | JP | 2003-19683 | A | |
| | | | | JP | 2003-52701 | A | |
| | | | | JP | 2003-61968 | A | |
| | | | | JP | 2003-61979 | A | |
| | | | | JP | 2003-79638 | A | |
| | | | | JP | 2003-127076 | A | |
| | | | | JP | 2003-127085 | A | |
| JP | 63-154187 | U1 | 11 October 1988 | (Family: none) | | | |
| JP | 61-142953 | U1 | 03 September 1986 | (Family: none) | | | |

Form PCT/ISA/210 (patent family annex) (January 2015)

**REFERENCES CITED IN THE DESCRIPTION**

**Patent documents cited in the description**

- WO 2017026167 A